# EUROPEAN PATENT SPECIFICATION

(11) **EP 0 974 374 B1**
(45) Date of publication and mention of the grant of the patent: **05.02.2003**
(21) Application number: 98910746.1
(22) Date of filing: 27.03.1998
(51) Int. Cl.: A61M 5/50, A61M 5/315, A61M 5/32

(54) **IMPROVED SYRINGE WITH INCORPORATED LOCKING SYSTEM OF THE SINGLE USE OR NON REUSABLE TYPE**
EINWEG- ODER NICHT WIEDERVERWENDBARE SPRITZE MIT EINGEBAUTEM BLOCKIERSYSTEM
SERINGUE PERFECTIONNEE DOTEE D'UN SYSTEME DE BLOCAGE INCORPORE, APPELEE JETABLE OU NON REUTILISABLE

(30) Priority: 14.11.1997 ES 9702399
(43) Date of publication of application: 26.01.2000
(73) Proprietor: Valle Sanchez, Manuel, 07610 Ca'n Pastilla-Mallorca (ES)
(72) Inventor: Valle Sanchez, Manuel, 07610 Ca'n Pastilla-Mallorca (ES)
(74) Representative: Liebetanz, Michael, Dipl.-Phys.
(86) International application number: ES9800079
(87) International publication number: WO99025403

(56) References cited:
- EP-A- 0 315 306
- WO-A-95/06490
- ES-U- 1 003 601
- FR-A- 2 658 724
- US-A- 3 834 387
- US-A- 5 030 208
- US-A- 5 122 124

## Description

The subject of the present invention is an improved syringe with a locking system incorporated, called single-use or non-reusable syringe.

As State of the Art it may be mentioned that, at the present time, syringes of this type are already known, which exhibit various locking systems which at times are very complex in their construction and which therefore make the said syringes expensive,

In other instances, the locking systems are so simple that they fail and the syringe can be reused. In this case the syringe does not fulfil the function of being non-reusable.

The locking system of the invention is simple and, that being so, economical to manufacture, but in addition it is effective since, once the syringe has been used, it cannot be reused because of its particular construction.

There is widespread awareness of the enormous problem posed by the reuse of a syringe which has been used by a sufferer from AIDS, an illness which is transmitted via blood, in that the virus of this illness is transmitted via needles and syringes in which small quantities of contaminated blood are present. This transmission is associated with drug addiction by the intravenous route.

Nowadays, the category with the most frequent wider transmission continues to be that of users by parenteral means.

It is obvious that, with the syringe of the invention, these serious problems produced by the reuse of syringes which are contaminated with the AIDS virus are avoided.

In accordance with the invention, the syringe comprises a cannula inside which a plunger is arranged so as to slide along an axial duct which opens out internally into a straight, widened section which the cannula features and which is extended into a frusto-conical tubing of smaller diameter into which the needle is fitted.

The plunger terminates, at its free inner end, in an elastic structure, such that, when it leaves the duct through which it slides and exits into the widened section of the cannula, it expands and thereupon takes up a larger diameter, which prevents it moving backwards and again being inserted into the internal duct of the cannula.

That is to say, reuse of the syringe is prevented, since the plunger remains inoperative.

The plunger, at its free inner end, exhibits an extended conical protrusion which terminates in a filiform end region, the protrusion length being designed such that when the plunger is located in the internal widened section of the cannula, the said protrusion has been fitted into the end portion of the injector needle, the widened conical protrusion and the injector needle remaining firmly joined together.

This constitutes one safety element of the syringe itself.

Another safety feature of the invention consists in equipping the interior of the cannula with a flexible perimetral rim which allows the plunger to pass in one direction and with a rim which the plunger exhibits when the end of the piston has exited into the widened section of the cannula, whereby, upon any attempt to move the plunger back in, locking is caused by having the inner rim of the cannula catch on the outer rim of the plunger.

Another safety feature which the syringe exhibits consists in that the needle is covered with a cap or top, establishing a dovetailed coupling which allows the top to be easily fitted but not removed due to the fact that the top has, at its entrance and internally, flexible structures which allow the needle to enter, as well as the free end of the cannula, but does not permit uncoupling in the opposite direction.

Finally, the syringe of the invention is arranged inside a packaging with a screw cap in order to protect the syringe and the needle.

With the objective of more easily understanding not only the construction but also the specific use of the syringe of the invention, a practical embodiment is related below, the said embodiment being purely illustrative and not in any way a limitation of the invention, all as shown in the attached drawings, in which:

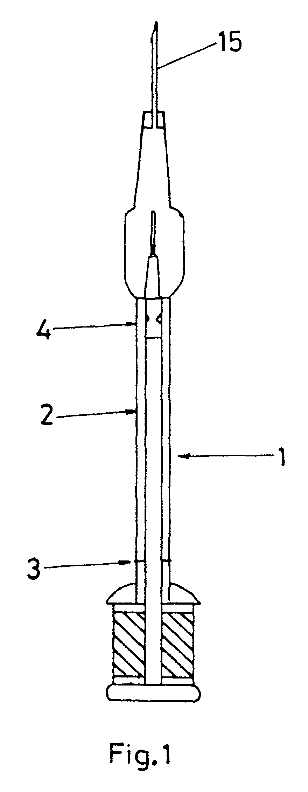

Figure 1 shows a sectioned elevation view of the syringe of the invention.
Figure 2 shows a side view of Figure 1.
Figure 3 shows a side view of the plunger of the syringe.
Figure 4 shows a detail of the coupling between the projecting end of the piston of the plunger and the end of the injector needle.
Figure 5 shows a view of one form of locking of the syringe.

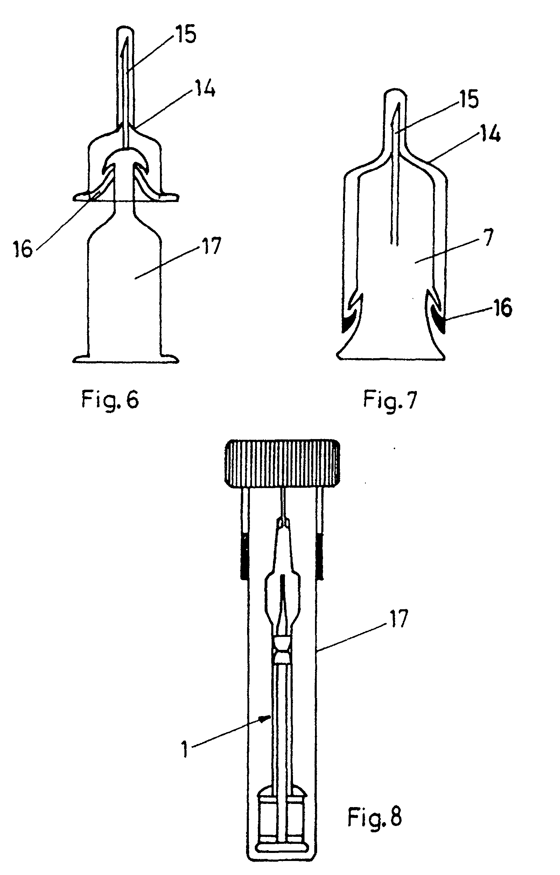

Figures 6 and 7 show details of the irremovable fixing of a top or cap protecting the needle.
Figure 8 shows a protective packaging for the syringe.

The syringe 1 comprises a cannula 2 through the inside of which a plunger 3 is fitted so that it can slide.

The plunger 3 terminates at its end in a widened section in the form of a piston 4, while the other end exhibits an expanded area 5 for actuating and pushing the actual piston 4.

An annular portion 6, which is of sufficient extent to prevent the expanded end 5 being moved in the positive-movement direction of the piston 4, is fitted in the plunger 3.

The piston 4 moves through the interior of a duct 6 of shorter length than that of the cannula 2.

The piston 4 exhibits flexible and elastic end regions and is equipped with an extended, conical, axial elongation 7 which blocks off and/or is fitted to the end 8 of the injector needle.

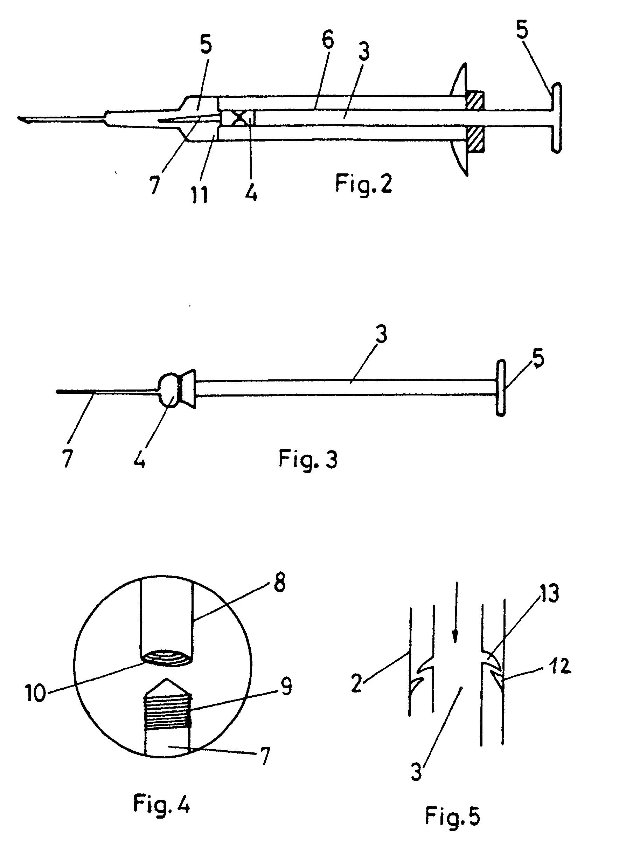

To that end, the axial elongation 7 exhibits a filiform region 9 which is fitted into another internal end region 10 of the end 8, Figure 4.

The abovementioned blocking occurs at the moment when the piston emerges into the widened region 11 of the cannula, and in addition to that, at that instant and position of the piston, it happens that an outer perimeteral ring 12 of the plunger has gone past an internal elastic perimetral projection 13 of the cannula, preventing the piston being moved back.

This same system is applied in the case of the use of a top or cap 14 for covering the needle 15 of the syringe, for which purpose the top, at its entry or opening exhibits a configuration in the form of a divided hood 16 which makes it possible for it to be fitted to the end of the end region of the axial elongation 7 of the cannula, while it prevents the top being removed in the opposite direction.

Once the syringe has been used the injector needle remains withdrawn inside the syringe.

The injector needle is fitted and/or mounted through the interior of the cannula, leaving its rear end 8 and 10 inside the tubing of the cannula.

These safety couplings are intended to be used after the syringe has been used.

Finally, the syringe is arranged, for sale, in a packaging 17 with a screw cap.

From this description, it can easily be understood that the invention protects a syringe which is simple not only in its construction but also in its handling, and, what is more important, the syringe is not reusable and is for single use.

## Claims

1. Syringe with locking system incorporated, called single-use or non-reusable syringe, comprising a cannula (2) and an internal plunger (3) with axial movement which terminates in a piston, which pressurizes and displaces the liquid to be injected by means of a needle fitted to a terminal tubing of the cannula, **characterized in that** the duct (6) through which the piston (4) of the plunger (3) moves is of a shorter length than that of the cannula (2), the said duct (6) delimiting a straight, widened, internal section , while the piston (4) consists of flexible and elastic end portions which, when they reach the said widened section, expand and take up a larger diameter which prevents the piston (4) from being able to move back in the opposite direction through the interior of the duct (6), resulting in the said syringe being non-reusable.

2. Syringe according to Claim 1, **characterized in that** the needle is protected by a cap or top (14) which has elastic means (16) available which allow the end of the cannula to pass but which prevent it exiting in the opposite direction.

3. Syringe according to Claim 1, **characterized in that** the plunger exhibits a perimetral protrusion (13) which, in its positive movement, overcomes the elastic resistance of an internal annular projection (12) which the cannula features, but which prevents the plunger moving back in the opposite direction.

4. Syringe according to Claim 1, **characterized in that** the plunger (3) exhibits an annular element of sufficient extent which prevents the positive movement of the plunger and prevents the piston (4) leaving its duct towards the internal widened section of the cannula.

## Patentansprüche

1. Spritze mit eingebautem Verschlusssystem, sogenannte Einweg- oder nicht wieder verwendbare Spritze, mit einer Kanüle (2) und einem internen Stössel (3) mit axialer Bewegung, die in einem Kolben endet, welche die Flüssigkeit unter Druck setzt und verschiebt, die mit Hilfe einer Nadel zu injizieren ist, die in einer Endröhre der Kanüle angeordnet ist, **dadurch gekennzeichnet, dass** die Leitung (6), durch welche sich der Kolben (4) des Stössels (3) bewegt, von kürzerer Länge ist als diejenige der Kanüle (2), wobei die besagte Leitung (6) einen geraden, verbreiterten inneren Abschnitt begrenzt, während der Kolben (4) aus biegsamen und elastischen Endabschnitten besteht, welche, wenn sie den besagten geweiteten Abschnitt erreichen, sich ausdehnen und einen grösseren Durchmesser aufnehmen, der verhindert, dass der Kolben (4) fähig ist, sich in die entgegengesetzte Richtung durch das Innere der Leitung (6) zurückzubewegen, was dazu führt, dass die besagte Spritze nicht wieder verwendbar ist.

2. Spritze nach Anspruch 1, **dadurch gekennzeichnet, dass** die Nadel durch eine Kappe oder Spitze (14) geschützt ist, die ein elastisches Mittel (16) aufweist, welches es erlaubt, dass das Ende der Kanüle hindurchtritt, aber dann verhindert, dass es in der entgegengesetzten Richtung austritt.

3. Spritze nach Anspruch 1, **dadurch gekennzeichnet, dass** der Stössel eine umkreisförmige Erhebung (13) aufweist, welche in ihrer positiven Bewegung den elastischen Widerstand einer inneren ringförmigen Erhebung (12) überwindet, den die Kanüle aufweist, aber dann verhindert, dass der Stössel zurück in die entgegengesetzte Richtung wandert.

4. Spritze nach Anspruch 1, **dadurch gekennzeichnet, dass** der Stössel (3) ein ringförmiges Element von ausreichender Grösse aufweist, das eine positive Bewegung des Stössels vermeidet und das ebenfalls vermeidet, dass der Kolben (4) seine Leitung zu dem inneren erweiterten Abschnitt der Kanüle verlässt.

## Revendications

1. Seringue dotée d'un système de blocage incorporé, appelée seringue jetable ou non réutilisable, comprenant une canule (2) et un plongeur interne (3) à mouvement axial se terminant dans un piston, qui met sous pression et déplace le liquide à injecter au moyen d'une aiguille montée sur un tube terminal de la canule, **caractérisée en ce que** le conduit (6) à travers lequel se déplace le piston (4) du plongeur (3) présente une longueur plus courte que celle de la canule (2), ledit conduit (6) délimitant une section interne élargie droite, tandis que le piston (4) est constitué de parties d'extrémité souples et élastiques qui, lorsqu'elles atteignent ladite section élargie, se dilatent et adoptent un diamètre plus grand empêchant le retour du piston (4) dans la direction opposée, à travers l'intérieur du conduit (6), si bien que ladite seringue est non-réutilisable.

2. Seringue selon la revendication 1, **caractérisée en ce que** l'aiguille est protégée par un embout ou un capuchon (14) disposant d'un moyen élastique (16) permettant le passage de l'extrémité de la canule mais l'empêchant de sortir dans la direction opposée.

3. Seringue selon la revendication 1, **caractérisé en ce que** le plongeur présente une protubérance périphérique (13) qui, dans son mouvement positif, surmonte la résistance élastique d'une saillie annulaire interne (12) dont est dotée la canule, mais qui empêche le retour du plongeur dans la direction opposée.

4. Seringue selon la revendication 1, **caractérisée en ce que** le plongeur (3) présente un élément annulaire d'étendue suffisante qui empêche le mouvement positif du plongeur et empêche le piston (4) de quitter son conduit en direction de la section interne élargie de la canule.
